# EUROPÄISCHE PATENTSCHRIFT

(11) **EP 4 291 258 B1**
(45) Veröffentlichungstag und Bekanntmachung des Hinweises auf die Patenterteilung: **01.01.2025**
(21) Anmeldenummer: 22708339.1
(22) Anmeldetag: 25.01.2022
(51) Int. Cl.: A61L 27/16, A61L 27/50, C08J 3/24, C08J 7/12, C08K 5/1545

(54) **VERFAHREN ZUR HERSTELLUNG EINES GLEITFLÄCHENELEMENTS, GLEITFLÄCHENELEMENT UND KNIEGELENKENDOPROTHESE**
METHOD FOR PRODUCING A SLIDING SURFACE ELEMENT, SLIDING SURFACE ELEMENT AND KNEE JOINT ENDOPROSTHESIS
PROCÉDÉ DE FABRICATION D'UN ÉLÉMENT DE SURFACE COULISSANT, ÉLÉMENT DE SURFACE COULISSANT ET ENDOPROTHÈSE D'ARTICULATION DU GENOU

(30) Priorität: 09.02.2021 DE 102021103016
(43) Veröffentlichungstag der Anmeldung: 20.12.2023
(73) Patentinhaber: Aesculap AG, 78532 Tuttlingen (DE)
(72) Erfinder: RICHTER, Berna, 78570 Mühlheim (DE); GRUPP, Thomas, 78588 Denkingen (DE); MULLIEZ, Marie Anne, 78532 Tuttlingen (DE); SCHWIESAU, Jens, 78532 Tuttlingen (DE); BOLLINGER, Arthur, 78532 Tuttlingen (DE)
(74) Vertreter: Hoeger, Stellrecht & Partner Patentanwälte mbB
(86) Internationale Anmeldenummer: PCT/EP2022/051594
(87) Internationale Veröffentlichungsnummer: WO 2022/171431

(56) Entgegenhaltungen:
- EP-A2- 1 795 212
- WO-A1-2018/046283
- WO-A2-2008/124825
- DE-A1- 102008 053 793
- US-A1- 2020 085 583

## Beschreibung

Die vorliegende Erfindung betrifft ein Verfahren zur Herstellung eines Gleitflächenelements auf Basis von UHMWPE für eine Gelenkendoprothese, insbesondere für eine Kniegelenkendoprothese.

Die Erfindung betrifft ferner ein Gleitflächenelement für eine Gelenkendoprothese, welches gemäß diesem Verfahren hergestellt ist.

Schließlich betrifft die Erfindung eine Kniegelenkendoprothese mit einer Femurkomponente und einer Tibiakomponente, wobei an der Tibiakomponente ein derartiges Gleitflächenelement festgelegt ist.

Es sind seit längerem Gelenkendoprothesen bekannt, die Gleitflächenelemente aus ultrahochmolekularem Polyethylen (UHMWPE) umfassen. Dabei wirkt das Gleitflächenelement aus UHMWPE in der Regel mit einem korrespondierenden Element aus einem metallischen Material zusammen, wobei sich mit dieser Materialkombination ein niedriger Reibungskoeffizient ergibt. Bei einer Hüftgelenkprothese ist das Gleitflächenelement aus UHMWPE typischerweise als Inlay des Hüftkopfimplantats ausgebildet, während es bei einer Kniegelenkprothese typischerweise an der Tibiakomponente festgelegt ist und auch als Meniskuselement bezeichnet wird. Auch bei einer Kniescheibenprothese (Patellaprothese) kann eine Gleitfläche aus UHMWPE eingesetzt werden.

Das Gleitflächenelement muss hinsichtlich seiner mechanischen Eigenschaften, Verschleißfestigkeit und Alterungsresistenz bestimmte Voraussetzungen erfüllen, wobei die hierfür erforderlichen Materialeigenschaften des UHMWPE zum Teil gegenläufig sind. Zur Erhöhung der Verschleißfestigkeit kann UHMWPE mit ionisierender Strahlung vernetzt werden, wie z.B. in der EP 0 995 450 A1 beschrieben. Gleichzeitig reduziert die Vernetzung jedoch die Duktalität des Materials, wodurch sich das Risiko einer strukturellen Materialermüdung, einer Delamination und gegebenenfalls eines Versagens des Gleitflächenelements erhöht.

Da bei der Strahlungsvernetzung von UHMWPE freie Radikale entstehen und teilweise in dem Material verbleiben, führt dies tendenziell zu einer schlechteren Alterungsresistenz aufgrund von oxidativen Prozessen. Diesem Problem kann mindestens teilweise durch den Zusatz von Antioxidantien wie zum Beispiel α-Tocopherol (Vitamin E) entgegengewirkt werden, wobei das Antioxidans entweder bereits vor der Herstellung eines Formkörpers dem UHMWPE zugesetzt wird, oder erst nach dem Vernetzen durch Diffusion in den Formkörper eingebracht wird (siehe zum Beispiel die EP 3 111 895 A1). Vor dem Hintergrund, dass zunehmend jüngere Patienten mit Gelenkendoprothesen versorgt werden, und dass gleichzeitig die Lebenserwartung steigt und die Patienten auch in höherem Alter aktiv bleiben, kommt der Alterungsresistenz von UHMWPE in Gelenkendoprothesen eine steigende Bedeutung zu.

Die Anforderungen an das Eigenschaftsprofil des UHMWPE unterscheiden sich auch je nach Art der Gelenkendoprothese. Bei einer Hüftgelenkprothese sind die zusammenwirkenden Gleitflächen im Wesentlichen kongruent (halbkugelförmig), so dass die auftretenden Kräfte mehr oder weniger gleichmäßig über die gesamte Kontaktfläche verteilt werden können. Daher spielt die Duktilität des Materials hier keine große Rolle. Ganz anders liegen die Verhältnisse bei einer Kniegelenkprothese, wo die zusammenwirkenden Gleitflächen weit weniger kongruent sind, um die gewünschte Bewegungsfreiheit des Gelenks (in der Sagittalebene und in der Frontalebene) verwirklichen zu können. Im Gegensatz zum Hüftgelenk finden hier simultan Gleit- und Rollbewegungen statt, wobei punktuelle Krafteinwirkungen und Spannungsspitzen auftreten, die bis zu einem Faktor 10 höher sind als bei der Hüfte. Bei Gleitflächenelementen aus hochvernetztem UHMWPE mit einer zu geringen Duktilität hat sich gezeigt, dass dies zu Rissen, Delaminationen und letztendlich zum Bruch und Versagen des Gleitflächenelements führen kann.

Die WO 2018/046283 A1 offenbart ein Verfahren zur Herstellung eines vernetzten Formkörpers aus UHMWPE, umfassend die Schritte: Bereitstellen eines Formkörpers aus UHMWPE, welches mit einem Antioxidans versetzt ist; Aufheizen des Formkörpers auf eine Temperatur von 100 °C oder mehr; und Bestrahlen des Formkörpers, um das UHMWPE in dem Formkörper zu vernetzen. Die Bestrahlung des Formkörpers erfolgt mit Röntgenstrahlung.

Die US 2020/085583 A1 offenbart eine implantierbare Knieprothese, die eine Femurkomponente, eine Tibiakomponente und eine Patellakomponente umfasst.

Der Erfindung liegt daher die Aufgabe zugrunde, ein Gleitflächenelement auf Basis von UHMWPE und ein Verfahren zu seiner Herstellung vorzuschlagen, welches in Bezug auf mechanische Festigkeit, Duktilität, Verschleißfestigkeit und Alterungsresistenz vorteilhafte Eigenschaften aufweist, und welches insbesondere in einer Kniegelenkendoprothese unter Berücksichtigung der erforderlichen Bewegungsfreiheit eingesetzt werden kann.

Diese Aufgabe wird gelöst durch ein Verfahren zur Herstellung eines Gleitflächenelements auf Basis von UHMWPE für eine Gelenkendoprothese, umfassend die Schritte:
- Mischen von UHMWPE in Pulverform mit 0,09 bis 0,11 Gew.% eines Antioxidans;
- Verdichten des mit Antioxidans versetzten UHMWPE zu einem Formkörper;
- Herstellen von einem oder mehreren Gleitflächenelementen aus dem Formkörper durch materialabtragende Bearbeitung; und
- Bestrahlen des Gleitflächenelements durch Gammastrahlung oder Röntgenstrahlung mit einer Strahlendosis von 25 bis 45 kGy, um das UHMWPE zu vernetzen,
wobei das Verfahren keine thermische Nachbehandlung des bestrahlten Gleitflächenelements umfasst.

Es hat sich überraschenderweise gezeigt, dass durch die Kombination der obigen Verfahrensparameter die Herstellung eines Gleitflächenelements mit einer hohen Verschleißfestigkeit, Alterungsresistenz, mechanischer Festigkeit und Duktilität möglich ist. Entscheidend ist dabei insbesondere die nur moderate Vernetzung des UHMWPE mit einer relativ geringen Strahlendosis unter Verwendung von Gammastrahlung oder Röntgenstrahlung, welche im Vergleich zu Betastrahlung eine wesentlich geringere Intensität und Absorption aufweisen. Ferner wird bei dem erfindungsgemäßen Verfahren keine thermische Nachbehandlung des bestrahlten Gleitflächenelements durchgeführt, die zu ungünstigen Materialveränderungen und Reduktion der Festigkeit führen kann. Durch den Entfall einer solchen Nachbehandlung ist das Verfahren auch kostengünstiger durchzuführen.

Das im Rahmen der Erfindung eingesetzte UHMWPE weist typischerweise ein Molekulargewicht im Bereich von 5·10⁶ bis 10⁷ g/mol auf (ermittelt aus der intrinsischen Viskosität) und eine Dichte im Bereich von 0,92 bis 0,95 g/cm³. Ein geeignetes UHMWPE in Pulverform ist z.B. von der Ticona GmbH unter der Bezeichnung GUR 1020 erhältlich, bzw. als Mischung mit 0,1 Gew.% α-Tocopherol als Antioxidans versetzt unter der Bezeichnung GUR 1020-E.

Als Antioxidans, das mit dem das UHMWPE gemischt wird, können verschiedene Antioxidationsmittel eingesetzt werden, die für eine Verwendung auch im medizinischen Bereich zugelassen sind. Bevorzugt ist das Antioxidans ausgewählt aus Tocopherolen, Tocotrienolen, Ascorbinsäure, polyphenolischen Antioxidantien wie z. B. Flavonoiden, Butylhydroxytoluol (BTH) und Butylhydroxyanisol (BTA).

Bei einer bevorzugten Ausführungsform der Erfindung ist das Antioxidans α-Tocopherol. Dieses wird auch als Vitamin E bezeichnet, wobei der Begriff Vitamin E in einem weiteren Sinne alle Tocopherole, Tocotrienole und weitere fettlösliche Antioxidantien umfasst.

Das Verdichten des UHMWPE zu einem Formkörper erfolgt typischerweise mittels Formpressen oder RAM-Extrusion, bevorzugt bis zu einer Dichte des Formkörpers von 0,92 g/cm³ oder mehr, d.h. im Wesentlichen bis zum Erreichen der theoretischen Dichte des UHMWPE. In der Regel weist der verdichtete Formkörper in jeder Raumrichtung eine Abmessung von 50 mm oder mehr auf, bevorzugt von 100 mm oder mehr, so dass aus diesem Rohling mehrere Gleitflächenelemente durch materialabtragende Bearbeitung hergestellt werden können, insbesondere mittels Fräsen.

Erfindungsgemäß wird dann die Bestrahlung des fertigen Gleitflächenelements mit seiner endgültigen Geometrie vorgenommen. Dadurch wird eine gleichmäßige Vernetzung insbesondere im Oberflächenbereich des Gleitflächenelements sichergestellt, im Gegensatz zu solchen Verfahren, bei denen zunächst ein Formkörper als Rohling bestrahlt wird und anschließend die Herstellung der jeweiligen Komponenten erfolgt.

Die Strahlendosis beträgt vorzugsweise 27 bis 33 kGy, und weiter bevorzugt ca. 30 kGy. Es hat sich gezeigt, das mit einer Strahlendosis in dieser Größenordnung ein optimaler Ausgleich zwischen den gegenläufigen Anforderungen, insbesondere der Verschleißfestigkeit und der Duktilität des Gleitflächenelements, verwirklicht werden kann.

Das Bestrahlen des Gleitflächenelements mit Gammastrahlung wird vorzugsweise über einen Zeitraum von 4 bis 6 h durchgeführt. Bei einer bevorzugten Strahlendosis von 30 kGy entspricht dies einer Dosisrate im Bereich von 5 bis 7,5 kGy/h.

Das Bestrahlen des Gleitflächenelements mit Röntgenstrahlung wird vorzugsweise über einen Zeitraum von 1 bis 10 min durchgeführt. Bei einer bevorzugten Strahlendosis von 30 kGy entspricht dies einer Dosisrate im Bereich von 0,05 bis 0,5 kGy/s.

Besonders günstig ist es, wenn das Gleitflächenelement vor dem Bestrahlen mit einer Kunststofffolie keimdicht verpackt wird. Neben der Vernetzung erfolgt durch die Gammastrahlung gleichzeitig eine Sterilisation des Gleitflächenelements in der hermetischen Verpackung.

Das Bestrahlen des Gleitflächenelements wird bevorzugt in einem Aufnahmeraum durchgeführt, der eine Gesamtbefüllung von bis zu 250 kg pro m³ aufweist.

Gegenstand der Erfindung ist ferner ein Gleitflächenelement auf Basis von UHMWPE für eine Gelenkendoprothese, das nach dem erfindungsgemäßen Verfahren hergestellt ist.

Die besonderen Vorteile und bevorzugten Ausführungsformen des erfindungsgemäßen Gleitflächenelements wurden bereits im Zusammenhang mit dem erfindungsgemäßen Verfahren erläutert.

Mit besonderem Vorteil ist das erfindungsgemäße Gleitflächenelement ein Gleitflächenelement für eine Kniegelenkendoprothese, insbesondere für eine Tibiakomponente oder Patellakomponente der Kniegelenendoprothese. In diesem Zusammenhang kann das Gleitflächenelement auch als Meniskuselement bezeichnet werden.

Die vorteilhaften mechanischen Eigenschaften des Gleitflächenelements, die sich aus der Anwendung des erfindungsgemäßen Herstellungsverfahrens ergeben, und die den Einsatz in einer Kniegelenkendoprothese möglich machen, lassen sich insbesondere anhand einem oder mehreren der folgenden Parameter charakterisieren:
Das Gleitflächenelement weist bevorzugt eine Bruchdehnung von 400% oder mehr auf, weiter bevorzugt von 450% oder mehr.

Das Gleitflächenelement weist bevorzugt eine Zugfestigkeit von 50 MPa oder mehr auf, weiter bevorzugt von 55 MPa.

Das Gleitflächenelement weist bevorzugt eine Streckspannung von 20 MPa oder mehr auf, insbesondere von 22 MPa oder mehr.

Das Gleitflächenelement weist bevorzugt eine Izod-Schlagzähigkeit von 100 kJ/m² oder mehr auf, weiter bevorzugt von 110 kJ/m² oder mehr.

Als Maß für den Vernetzungsgrad von UHMWPE kann das Quellverhältnis herangezogen werden, welches mit zunehmender Vernetzung abnimmt. Das erfindungsgemäße Gleitflächenelement weist bevorzugt ein Quellverhältnis von über 4 auf, resultierend aus der nur moderaten Vernetzung in dem erfindungsgemäßen Verfahren. Das Quellverhältnis wird hierbei gemäß der Norm ASTM F 2214:2016 ermittelt (Bestimmung der Volumenänderung des vernetzten UHMWPE durch Quellen in o-Xylol).

Ein weiterer Gegenstand der vorliegenden Erfindung ist eine Kniegelenkendoprothese, umfassend eine Femurkomponente und eine Tibiakomponente, wobei an der Tibiakomponente ein erfindungsgemäßes Gleitflächenelement festgelegt ist, das mit der Femurkomponente zusammenwirkt. Dabei liegen die Radienverhältnisse der Oberflächenbereiche der Femurkomponente und des Gleitflächenelements, die über den Bewegungsumfang der Kniegelekendoprothese miteinander in Kontakt kommen, im Bereich von 1 bis 7.

Der weite Bereich von Radienverhältnissen bei der erfindungsgemäßen Kniegelenkendoprothese verdeutlicht die geringe Kongruenz (z.B. im Vergleich zu einer Hüftgelenkprothese) zwischen der Femurkomponente und dem Gleitflächenelement, die bei einer Kniegelenkprothese erforderlich ist, um die gewünschte Bewegungsfreiheit bei der Betätigung des Gelenks in der Sagittalebene und in der Frontalebene zu verwirklichen.

Die bei der erfindungsgemäßen Kniegelenkendoprothese auf das Gleitflächenelement wirkenden Druckspannungen, insbesondere Punktbelastungen, liegen typischerweise bei bis zu 25 MPa. Aufgrund der mechanischen Eigenschaften des erfindungsgemäßen Gleitflächenelements sind diese Werte unkritisch.

Die Erfindung wird anhand der nachfolgenden Beispiele unter Bezugnahme auf die Figuren näher erläutert. Es zeigen:
- Figur 1:: Ein Netzdiagramm zum Eigenschaftsprofil verschiedener UHMWPE-Materialien; und
- Figur 2:: ein Diagramm zum Druckspannungsverlauf bei einer Kniegelenkendoprothese mit einem erfindungsgemäßen Gleitflächenelement.

### Eigenschaftsprofil von vernetztem UHMWPE

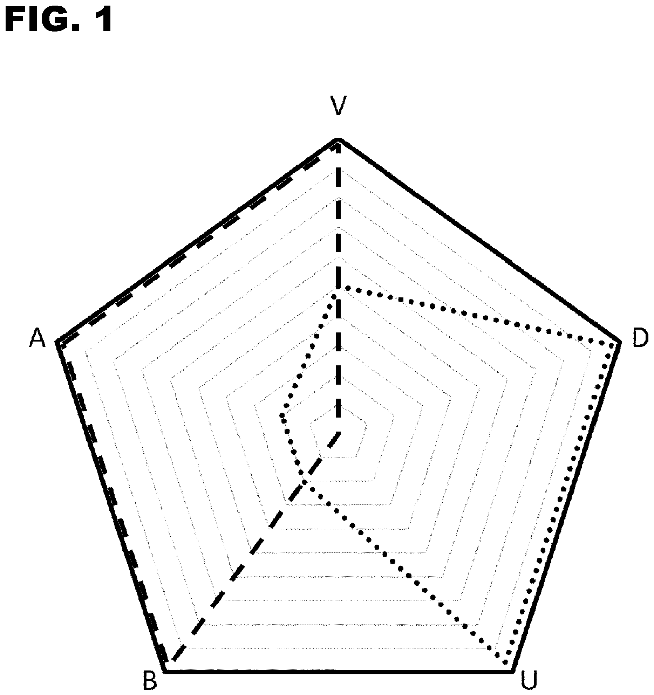

Das Netzdiagramm in der Figur 1 zeigt schematisch das typische Eigenschaftsprofil des moderat vernetzten UHMWPE des erfindungsgemäßen Gleitflächenelements (durchgehende Linie), eines hochvernetzten UHMWPE mit Zusatz von Vitamin E (gestrichelte Linie), und eines Standard-UHMWPE ohne Zusatz von Vitamin E (gepunktete Linie).

Die fünf Achsen des Diagramms repräsentieren folgende Eigenschaften der Materialien:
- V:: Verschleißfestigkeit
- D:: Duktilität
- U:: Unempfindlichkeit gegen Spannungsspitzen
- B:: Bewegungsfreiheit (durch geringe Kongruenz im Gelenk)
- A:: Alterungsresistenz

Das Diagramm zeigt schematisch, dass mit dem erfindungsgemäßen Gleitflächenelement alle geforderten Eigenschaften in einem hohen Ausmaß erfüllt werden können, während dies mit den UHMWPE-Materialien aus dem Stand der Technik nicht möglich ist, insbesondere aufgrund der gegenläufigen Effekte eines hohen Vernetzungsgrades. Dieser ermöglicht zwar eine hohe Verschleißfestigkeit und Alterungsresistenz, verringert aber auf der anderen Seite die Duktilität des Materials und macht es empfindlich gegen Spannungsspitzen. Zudem ermöglicht das erfindungsgemäße Gleitflächenelement eine besonders hohe kinematische Bewegungsfreiheit der Gelenkendoprothese, indem es den hohen Punktbelastungen standhält, die bei wenig kongruenten Gelenkpartnern auftreten.

### Beispiel

Zur Herstellung eines erfindungsgemäßen Gleitflächenelements wurde pulverförmiges UHMWPE (GUR 1020, Ticona GmbH) mit 0,1 Gew.% Vitamin E (α-Tocopherol) homogen vermischt und zu einer Platte verpresst. Aus diesem Rohling wurde ein Gleitflächenelement für eine Tibiakomponente einer Kniegelenkendoprothese ausgefräst. Nach dem Verpacken in Folie wurde das Gleitflächenelement für ca. fünf Stunden mit Gammastrahlung bestrahlt, um das UHMWPE zu vernetzen und gleichzeitig zu sterilisieren. Die Strahlendosis betrug hierbei 30 ± 3 kGy.

In der nachfolgenden Tabelle sind die relevanten mechanischen Eigenschaften des erfindungsgemäß hergestellten Gleitflächenelements angegeben, sowie zum Vergleich die entsprechenden Werte (aus der Literatur) für kommerziell erhältliche, vernetzte UHMWPE-Materialien:

| | Bruchdehnung | Zugfestigkeit | Streckspannung | Izod-Schlagzähigkeit |
|---|---|---|---|---|
| Erfindung | 457±9% | 59,7±2,9 MPa | 22,4±0,1 MPa | 119,4±2,2 kJ/m² |
| XLPE (Smith & Nephew) | 300±20% | 56±7,1 MPa | 20±1,3 MPa | k.A. |
| Marathon (DePuy/J and J) | 290±14% | 56±5,7 MPa | 21±1,5 MPa | k.A. |
| Crossfire (Stryker Howmedica) | 280±37% | 48±7,2 MPa | 24±1,3 MPa | k.A. |

Der Vergleich zeigt, dass das erfindungsgemäße Gleitflächenelement dem Stand der Technik insbesondere bei der Bruchdehnung deutlich überlegen ist.

Auch die Zugfestigkeit des erfindungsgemäßen UHMWPE ist höher als bei allen drei bekannten Materialien.

Zur Ermittlung der Belastungen in einer Kniegelenkendoprothese wurde das erfindungsgemäße Gleitflächenelement als Meniskuskomponente mit einer Femurkomponente kombiniert, wobei die Radienverhältnisse der Oberflächenbereiche, die über den Bewegungsumfang des Gelenks miteinander in Kontakt kommen, im Bereich von 1 bis 7 liegen.

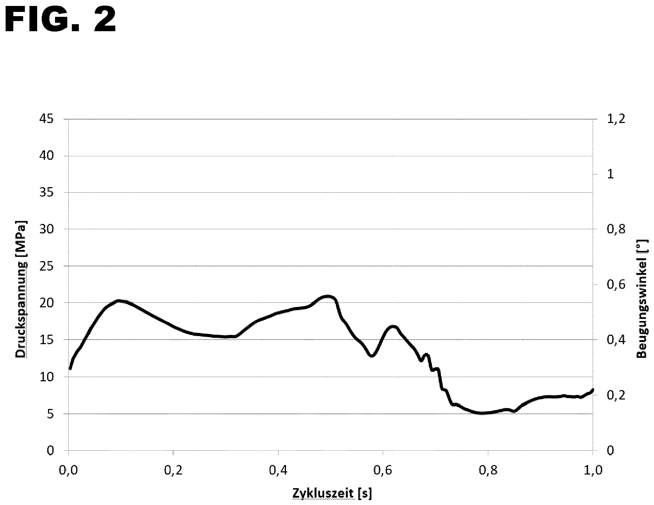

Der Verlauf der Druckspannung zwischen dem Gleitflächenelement und der Femurkomponente über eine Zykluszeit von einer Sekunde ist in der Figur 2 dargestellt. Die Werte liegen durchgehend unter 25 MPa, und sind im Hinblick auf die mechanischen Eigenschaften des erfindungsgemäßen Gleitflächenelements unkritisch.

## Patentansprüche

1. Verfahren zur Herstellung eines Gleitflächenelements auf Basis von UHMWPE für eine Gelenkendoprothese, umfassend die Schritte:
- Mischen von UHMWPE in Pulverform mit 0,09 bis 0,11 Gew.% eines Antioxidans;
- Verdichten des mit dem Antioxidans versetzten UHMWPE zu einem Formkörper;
- Herstellen von einem oder mehreren Gleitflächenelementen aus dem Formkörper durch materialabtragende Bearbeitung; und
- Bestrahlen des Gleitflächenelements durch Gammastrahlung oder Röntgenstrahlung mit einer Strahlendosis von 25 bis 45 kGy, um das UHMWPE zu vernetzen,
wobei das Verfahren keine thermische Nachbehandlung des bestrahlten Gleitflächenelements umfasst.

2. Verfahren nach Anspruch 1, wobei das UHMWPE ein Molekulargewicht im Bereich von 5*10⁶ bis 10⁷ g/mol und eine Dichte im Bereich von 0,92 bis 0,95 g/cm³ aufweist.

3. Verfahren nach Anspruch 1 oder 2, wobei das Antioxidans ausgewählt ist aus Tocopherolen, Tocotrienolen, Ascorbinsäure, polyphenolischen Antioxidantien wie z.B. Flavonoiden, Butylhydroxytoluol und Butylhydroxyanisol.

4. Verfahren nach Anspruch 3, wobei das Antioxidans α-Tocopherol ist.

5. Verfahren nach einem der vorhergehenden Ansprüche, wobei das mit dem Antioxidans versetzte UHMWPE mittels Formpressen oder RAM-Extrusion verdichtet wird, bevorzugt bis zu einer Dichte des Formkörpers von 0,92 g/cm³ oder mehr.

6. Verfahren nach einem der vorhergehenden Ansprüche, wobei die Strahlendosis 27 bis 33 kGy beträgt, bevorzugt ca. 30 kGy.

7. Verfahren nach einem der vorhergehenden Ansprüche, wobei das Bestrahlen des Gleitflächenelements mit Gammastrahlung über einen Zeitraum von 4 bis 6 h oder mit Röntgenstrahlung über einen Zeitraum von 1 bis 10 min durchgeführt wird.

8. Verfahren nach einem der vorhergehenden Ansprüche, wobei das Gleitflächenelement vor dem Bestrahlen in einer Kunststofffolie keimdicht verpackt wird.

9. Verfahren nach einem der vorhergehenden Ansprüche, wobei das Bestrahlen des Gleitflächenelements in einem Aufnahmeraum durchgeführt wird, der eine Gesamtbefüllung von bis zu 250 kg pro m³ aufweist.

10. Gleitflächenelement auf Basis von UHMWPE für eine Gelenkendoprothese, das gemäß dem Verfahren nach einem der vorhergehenden Ansprüche hergestellt ist.

11. Gleitflächenelement nach Anspruch 10, wobei das Gleitflächenelement ein Gleitflächenelement für eine Kniegelenkendoprothese ist, insbesondere für eine Tibiakomponente oder Patellakomponente der Kniegelenkendoprothese.

12. Gleitflächenelement nach Anspruch 10 oder 11, wobei das Gleitflächenelement eine Bruchdehnung von 400% oder mehr aufweist, bevorzugt von 450% oder mehr.

13. Gleitflächenelement nach einem der Ansprüche 10 bis 12, wobei das Gleitflächenelement eine Zugfestigkeit von 50 MPa oder mehr aufweist, bevorzugt von 55 MPa oder mehr.

14. Gleitflächenelement nach einem der Ansprüche 10 bis 13, wobei das Gleitflächenelement eine Streckspannung von 20 MPa oder mehr aufweist, bevorzugt von 22 MPa oder mehr; und/oder
wobei das Gleitflächenelement eine Izod-Schlagzähigkeit von 100 kJ/m² oder mehr aufweist, bevorzugt von 110 kJ/m² oder mehr; und/oder
wobei das Gleitflächenelement ein Quellverhältnis von über 4 aufweist, ermittelt gemäß ASTM F 2214:2016.

15. Kniegelenkendoprothese, umfassend eine Femurkomponente und eine Tibiakomponente, wobei an der Tibiakomponente ein Gleitflächenelement nach einem der Ansprüche 10 bis 14 festgelegt ist, das mit der Femurkomponente zusammenwirkt,
wobei die Radienverhältnisse der Oberflächenbereiche der Femurkomponente und des Gleitflächenelements, die über den Bewegungsumfang der Kniegelenkendoprothese miteinander in Kontakt kommen, im Bereich von 1 bis 7 liegen.

## Claims

1. Method for producing a sliding surface element on the basis of UHMWPE for a joint endoprosthesis, comprising the steps:
- mixing UHMWPE in powder form with 0.09 to 0.11% by weight of an antioxidant;
- compacting the UHMWPE mixed with the antioxidant into a molded body;
- manufacturing one or more sliding surface elements from the molded body by material-removing machining; and
- irradiating the sliding surface element by gamma radiation or X-ray radiation with a radiation dose of 25 to 45 kGy in order to crosslink the UHMWPE,
wherein the method comprises no thermal post-treatment of the irradiated sliding surface element.

2. Method in accordance with Claim 1, wherein the UHMWPE has a molecular weight in the range of 5*10⁶ to 10⁷ g/mol and a density in the range of 0.92 to 0.95 g/cm³.

3. Method in accordance with Claim 1 or 2, wherein the antioxidant is selected from tocopherols, tocotrienols, ascorbic acid, polyphenolic antioxidants like, e.g., flavonoids, butylhydroxytoluol, and butylhydroxyanisole.

4. Method in accordance with Claim 3, wherein the antioxidant is α-tocopherol.

5. Method in accordance with any one of the preceding Claims, wherein the UHMWPE mixed with the antioxidant is compressed by means of compression molding or RAM extrusion, preferably up to a density of the molding body of 0.92 g / cm³ or more.

6. Method in accordance with any one of the preceding Claims, wherein the radiation dose is 27 to 33 kGy, preferably about 30 kGy.

7. Method in accordance with any one of the preceding Claims, wherein the irradiation of the sliding surface element is performed with gamma radiation over a period of 4 to 6 h or with X-ray radiation over a period of 1 to 10 min.

8. Method in accordance with any one of the preceding Claims, wherein the sliding surface element is packaged in a plastic film in a germ-tight manner before irradiation.

9. Method in accordance with any one of the preceding Claims, wherein the irradiation of the sliding surface element is performed in a receiving space that has a total filling of up to 250 kg per m³.

10. Sliding surface element on the basis of UHMWPE for a joint endoprosthesis, which is produced according to the method in accordance with any one of the preceding Claims.

11. Sliding surface element in accordance with Claim 10, wherein the sliding surface element is a sliding surface element for a knee joint endoprosthesis, in particular for a tibial component or patella component of the knee joint endoprosthesis.

12. Sliding surface element in accordance with Claim 10 or 11, wherein the sliding surface element has an elongation at break of 400% or more, preferably of 450% or more.

13. Sliding surface element in accordance with any one of Claims 10 to 12, wherein the sliding surface element has a tensile strength of 50 MPa or more, preferably of 55 MPa or more.

14. Sliding surface element in accordance with any one of Claims 10 to 13, wherein the sliding surface element has a yield stress of 20 MPa or more, preferably of 22 MPa or more; and/or wherein the sliding surface element has an Izod impact strength of 100 kJ/m² or more, preferably of 110 kJ/m² or more; and/or wherein the sliding surface element has a swelling ratio of over 4, determined in accordance with ASTM F 2214:2016.

15. Knee joint endoprosthesis, comprising a femoral component and a tibial component, wherein a sliding surface element in accordance with any one of Claims 10 to 14 is fixed to the tibial component and cooperates with the femoral component, wherein the radii ratios of the surface regions of the femoral component and the sliding surface element that come into contact with one another over the range of motion of the knee joint endoprosthesis are in the range of 1 to 7.

## Revendications

1. Procédé de fabrication d'un élément surface de glissement à base de UHMWPE pour une endoprothèse d'articulation, comprenant les étapes suivantes :
- mélange de UHMWPE sous forme de poudre avec 0,09 à 0,11 % en poids d'un antioxydant ;
- compression du UHMWPE avec l'antioxydant, afin de former un corps moulé ;
- fabrication d'un ou plusieurs éléments à surface de glissement à partir du corps moulé par usinage par enlèvement de matériau ; et
- irradiation de l'élément à surface de glissement par un rayonnement gamma ou un rayonnement X avec une dose de rayonnement de 25 à 45 kGy, afin de réticuler le UHMWPE,
dans lequel le procédé ne comprend aucun post-traitement thermique de l'élément à surface de glissement irradié.

2. Procédé selon la revendication 1, dans lequel le UHMWPE présente un poids moléculaire de l'ordre de 5*10⁶ à 10⁷ g/mol et une densité de l'ordre de 0,92 à 0,95 g/cm³.

3. Procédé selon la revendication 1 ou 2, dans lequel l'antioxydant est sélectionné dans le groupe suivant : tocophéroles, tocotriénoles, acide ascorbique, antioxydants polyphénoliques, par exemple des flavonoïdes, du butylhydroxytoluol et le butylhydroxyanisol.

4. Procédé selon la revendication 3, dans lequel l'antioxydant est du α-tocophérole.

5. Procédé selon l'une des revendications précédentes, dans lequel le UHMWPE additionné d'antioxydant est comprimé par pressage ou par extrusion RAM, de préférence jusqu'à une densité du corps moulé de 0,92 g/cm³ ou plus.

6. Procédé selon l'une des revendications précédentes, dans lequel la dose de rayonnement est de 27 à 33 kGy, de préférence d'environ 30 kGy.

7. Procédé selon l'une des revendications précédentes, dans lequel l'irradiation de l'élément à surface de glissement est effectuée avec un rayonnement gamma sur une période de 4 à 6 h ou avec un rayonnement X sur une période de 1 à 10 min.

8. Procédé selon l'une des revendications précédentes, dans lequel l'élément à surface de glissement est emballé de manière stérile dans un film de matière plastique avant l'irradiation.

9. Procédé selon l'une des revendications précédentes, dans lequel l'irradiation de l'élément à surface de glissement est effectuée dans un compartiment de logement qui présente un remplissage total jusqu'à 250 kg par m³.

10. Élément à surface de glissement à base de UHMWPE pour une endoprothèse d'articulation, qui est fabriqué à l'aide du procédé selon l'une des revendications précédentes.

11. Élément à surface de glissement selon la revendication 10, dans lequel l'élément à surface de glissement est un élément de surface de glissement pour une endoprothèse d'articulation de genou, plus particulièrement pour un composant de tibia ou de rotule de l'endoprothèse d'articulation de genou.

12. Élément à surface de glissement selon la revendication 10 ou 11, dans lequel l'élément à surface de glissement présente un allongement à la rupture de 400 % ou plus, de préférence de 450 % ou plus.

13. Élément à surface de glissement selon l'une des revendications 10 à 12, dans lequel l'élément à surface de glissement présente une résistance à la traction de 50 MPa ou plus, de préférence de 55 MPa ou plus.

14. Élément à surface de glissement selon l'une des revendications 10 à 13, dans lequel l'élément à surface de glissement présente une résistance à l'étirage de 20 MPa ou plus, de préférence de 22 MPa ou plus ; et/ou dans lequel l'élément à surface de glissement présente une résistance à l'impact Izod de 100 kJ/m² ou plus, de préférence de 110 kJ/m² ou plus ; et/ou dans lequel l'élément à surface de glissement présente un taux de gonflement supérieur à 4, déterminé selon ASTM F 2214:2016.

15. Endoprothèse d'articulation de genou comprenant un composant de fémur et un composant de tibia, dans lequel, sur le composant de tibia, est fixé un élément à surface de glissement selon l'une des revendications 10 à 14, qui interagit avec le composant de fémur,
dans lequel les rapports des rayons des zones superficielles du composant de fémur et de l'élément à surface de glissement, qui entrent en contact entre elles sur l'étendue du mouvement de l'endoprothèse d'articulation de genou, sont de l'ordre de 1 à 7.
